Europäisches Patentamt

European Patent Office

Office européen des brevets

(19)

(11) Numéro de publication : **0 454 599 B1**

(12) **FASCICULE DE BREVET EUROPEEN**

(45) Date de publication du fascicule du brevet :
27.07.94 Bulletin 94/30

(51) Int. Cl.$^5$ : **A61L 27/00, A61F 2/08**

(21) Numéro de dépôt : **91420137.1**

(22) Date de dépôt : **25.04.91**

(54) **Prothèse ligamentaire et procédé de fabrication.**

(30) Priorité : **25.04.90 FR 9005942**

(43) Date de publication de la demande :
**30.10.91 Bulletin 91/44**

(45) Mention de la délivrance du brevet :
**27.07.94 Bulletin 94/30**

(84) Etats contractants désignés :
**AT BE CH DE DK ES FR GB GR IT LI LU NL SE**

(56) Documents cités :
**EP-A- 0 296 078**

(73) Titulaire : **SA ICP FRANCE**
**Boulevard du Maréchal Juin**
**F-52000 Chaumont (FR)**
Titulaire : **Michel, Jean-Pierre**
**Rochetaillée**
**F-52210 Arc-en-Barrois (FR)**

(72) Inventeur : **Gagnieu, Christian**
**19 rue Pierre et Marie Curie**
**F-69680 Chassieu (FR)**
Inventeur : **Damour, Odile**
**32 rue Charles Luizet**
**F-69230 Saint-Genis-Laval (FR)**
Inventeur : **Vedel, François**
**La lézardière,**
**Chemin de Répentance**
**F-13100 Aix en Provence (FR)**
Inventeur : **Echinard, Christian**
**Mas de la Baume**
**F-13420 Gemenos (FR)**

(74) Mandataire : **Guerre, Dominique et al**
**Cabinet Germain et Maureau**
**20 Boulevard Eugène Deruelle**
**BP 3011**
**F-69392 Lyon Cédex 03 (FR)**

## Description

**Prothèse ligamentaire et procédé de fabrication**

L'invention concerne une prothèse ligamentaire à usage humain ou vétérinaire, pour remplacer un ligament naturel.

Les prothèses actuellement décrites font office de ligaments artificiels destinés à remplacer ou à renforcer les ligaments lésés.

Elles sont réalisées à partir d'une armature en matériaux synthétiques tolérés par l'organisme et présentant une certaine résistance à la traction avec une capacité d'extension limitée, par élasticité notamment. A titre d'exemple de matériaux synthétiques, on peut citer les fibres de polytéréphtaéthylène-glycol commercialisées sous la marque DACRON, les fibres polytétrafluoroéthylèniques connues sous la marque TEFLON, et les fibres de polyamide (NYLON).

Les prothèses ligamentaires se heurtent à deux problèmes majeurs, le premier mécanique, le second biologique, c'est-à-dire respectivement :
- le problème de la rupture, la prothèse étant sans cesse soumise à des flexions, extensions et abrasions sur les crêtes osseuses
- le problème des réactions d'intolérance, malgré la biocompatibilité des matériaux employés, entraînant des synovites, voire des rejets.

Une solution au premier problème est notamment apportée par des prothèses réalisées par le tricotage d'un fil polyéthylènique de grande résistance, commercialisé sous la marque RASHEL par exemple, conférant au treillis ainsi obtenu, souplesse et solidité.

Le second problème est lié à une habitation ou colonisation de la prothèse ligamentaire consistant en l'obtention d'un tissu collagénique, fibroplastique ou conjonctif. Les prothèses habitables actuellement existantes, parfois réalisées en un matériau biodégradable, n'induisent pas une colonisation de bonne qualité, ce qui entraîne des complications immunologiques et/ou mécaniques.

Le document EP-A-0 296 078 décrit un biomatériau à base de l'association collagène-chitosane-glycosaminoglycanes colonisable, notamment par les fibroplastes, et propose d'utiliser un tel biomatériau pour couvrir la prothèse ligamentaire et ainsi en faciliter son habitation cellulaire.

L'interface entre la prothèse et le tissu environnant est constitué par une matrice extracellulaire, collagène-chitosane-glycosaminoglycanes, facilitant le développement de fibroplastes et la reconstitution du tissu conjonctif.

Cependant, cette couche de matrice extracellulaire d'une part est biodégradable au risque de disparaître avant que l'habitation n'ait substantiellement avancé, d'autre part ne permet qu'une colonisation extérieure en surface de la prothèse.

La présente invention apporte une prothèse ligamentaire qui résoud à la fois les problèmes mécaniques et biologiques rencontrés par les prothèses connues.

La prothèse selon l'invention consiste essentiellement en une armature allongée, souple et résistante et en une matrice extracellulaire couvrant ladite armature, l'armature étant une gaine tubulaire perméable à la matrice extracellulaire à l'état liquide, ladite gaine étant remplie avec une matrice extracellulaire interne sèche et recouverte à l'extérieur par une matrice extracellulaire externe sèche, la gaine étant traversée par l'une et/ou l'autre des deux matrices extracellulaires.

Par matrice extracellulaire dans la présente invention, on entend un matériau extracellulaire biologique artificiel, à l'origine à l'état liquide et à l'état solide, poreux, après déshydratation.

Dans la prothèse selon la présente invention, la résistance mécanique est procurée par l'armature, et les performances biologiques, pour la reconstitution d'un ligament normal, sont apportées par la matrice extracellulaire qui disposée de part et d'autre de l'armature la remplit et la traverse complètement, rendant la colonisation quantitativement et qualitativement viable.

La gaine est perméable à la matrice extracellulaire liquide pour faciliter l'interpénétration des deux matrices extracellulaires noyant ainsi le réseau de la gaine pour ensuite favoriser la migration des fibroplastes d'une matrice extracellulaire à l'autre.

Selon une réalisation préférentielle de l'invention, la gaine est constituée par des fils synthétiques tissés, tels que le polytéréphtaéthylène-glycol, tressés ou tricotés.

Les matrices extracellulaires dont la nature est choisie pour favoriser la migration, la multiplication et la croissance des fibroplastes sont constituées par un matériau extracellulaire biologique artificiel, qui selon une réalisation préférentielle de l'invention est à base de l'association collagène-chitosane-glycosaminoglycanes.

Selon cette réalisation, les matrices extracellulaires comprennent du collagène pour 60 à 80 %, choisi parmi les collagènes de type I à XV d'origine humaine ou animale, natifs ou sans télopeptide, par du chitosane pour 10 à 30 %, le chitosane provenant de la N-désacétylation de la chitine d'invertébrés et notamment de crabes et par des glycosaminoglycanes pour 6 à 10 %, choisis parmi l'acide hyaluronique, la chondroïtine-4-sulfate, la chondroïtine-6-sulfate, le dermatane-sulfate, l'héparane-sulfate, le kératane-sulfate et l'héparine, ces pourcentages représentent une concentration pondérale par rapport au poids sec de la matrice.

Pour d'autres réalisations particulières de prothèse selon cette invention, les matrices extracellulaires peuvent être choisies parmi les matériaux suivants : collagènes réticulés et dérivés, chitosanes réacétylés et dérivés, chitosanes réticulés et dérivés, combinaison collagène réticulé et chitosane réticulé, collagène réticulé avec un chitosane, combinaison collagène réticulé et glycosaminoglycanes réticulés.

Les matériaux constitutifs de chacune des matrices extracellulaires interne et externe peuvent être respectivement les mêmes en proportions identiques ou non ou bien différents.

Le but de la présente invention est également un procédé pour réaliser une telle prothèse, c'est-à-dire obtenir une parfaite imprégnation de la gaine tubulaire par les deux matrices extracellulaires interne et externe et une bonne cohésion de la prothèse ainsi obtenue.

La présente invention propose plus particulièrement deux procédés de fabrication d'une prothèse : un premier plus spécifiquement adapté pour réaliser des prothèses de faible diamètre et le second préférentiellement adapté pour réaliser des prothèses de diamètre plus élevé.

L'un des procédés peut par exemple être utilisé pour un diamètre de gaine notamment inférieur à environ 9 mm, tandis que l'autre procédé peut être utilisé pour un diamètre supérieur à environ 9 mm. On aborde ci-après chacun des deux procédés, étant bien évident que les valeurs numériques concernant le diamètre de la gaine évaluées expérimentalement, sont données à titre indicatif nullement limitatif.

Pour la réalisation d'une prothèse ligamentaire notamment inférieure à 9 mm, on procède selon les étapes suivants :
- on introduit la gaine dans un tube ouvert à ses deux extrémités
- on introduit la matrice extracellulaire liquide par l'une des extrémités du tube pour assurer une imprégnation homogène de la gaine
- on congèle l'ensemble
- on déshydrate l'ensemble dans le tube ou après démoulage.

Pour la réalisation de prothèses de diamètre plus élevé et plus spécifiquement supérieur à 9 mm, le procédé décrit ci-dessus présente certains inconvénients liés au volume plus grand de matrice extracellulaire liquide à manipuler. En effet, d'une part, après son introduction dans le tube et la gaine, la matrice extracellulaire liquide peut se répandre par les extrémités ouvertes du tube, et d'autre part, au cours de la congélation on risque de ne pas congeler toute la matrice extracellulaire, et en particulier le coeur de la matrice extracellulaire interne.

Ainsi, dans le cas d'une prothèse de diamètre d'environ 9 mm et plus, le procédé préférentiel comprend les principales étapes suivantes :
- après préparation de la matrice extracellulaire interne liquide, on la coule dans un tube cylindrique et on la congèle pendant un temps déterminé apte à conférer une température stable au sein du produit
- on déshydrate la matrice extracellulaire interne dans le tube ou après démoulage
- on introduit la matrice extracellulaire interne déshydratée dans la gaine
- on plonge la gaine contenant la matrice extracellulaire déshydratée dans un tube contenant une quantité de matrice extracellulaire externe liquide pour imprégner ladite gaine dans sa totalité et on congèle l'ensemble après un temps de contact déterminé
- on déshydrate l'ensemble dans le tube ou après démoulage.

L'invention est à présent décrite en référence aux figures annexées, selon lesquelles :
- Figure 1 repésente une vue en coupe longitudinale d'une prothèse selon l'invention
- Figure 2 représente une vue en perspective partiellement arrachée d'une prothèse montrant successivement la matrice extracellulaire interne 2′, le support ou gaine 1, la matrice extracellulaire externe 2.
- Figure 3 est une copie de photographie d'une prothèse selon l'invention, vue en coupe en microscope électronique à balayage
- Figure 4 est une copie de photographie d'une matrice extracellulaire collagène 72 %-chitosane 20 %-chondroïtine-4-et-6-sulfate 8 %, vue en coupe en microscope électronique.

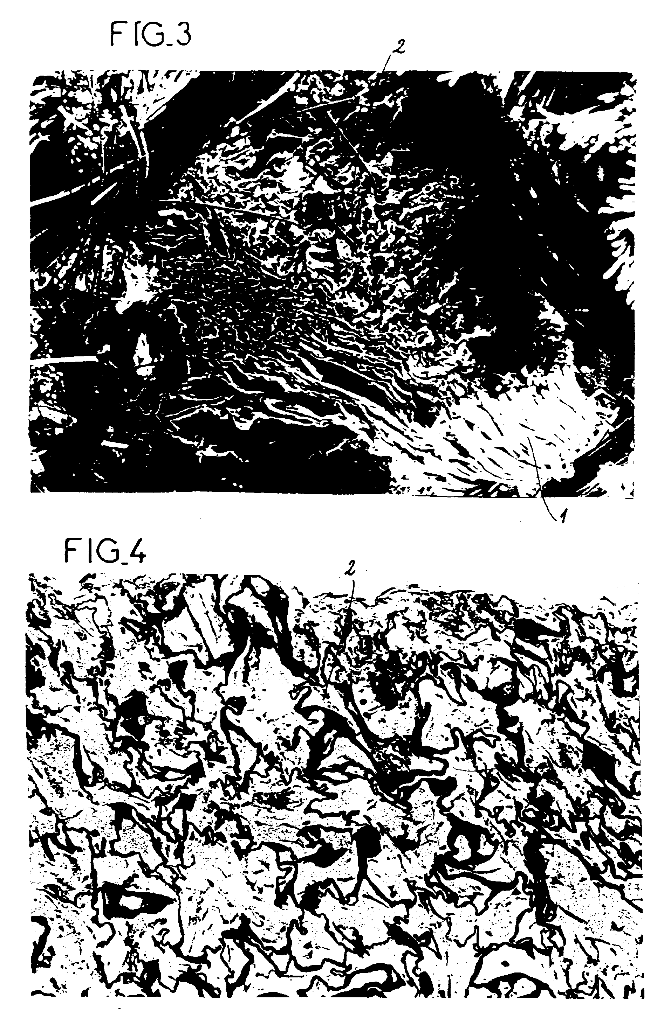

Selon l'invention, la prothèse ligamentaire est constituée par la combinaison d'au moins deux matériaux biocompatibles montrés en Figure 3 respectivement sous forme d'un support ou gaine 1 et de deux matrices extracellulaires interne 2′ et externe 2, biodégradables et déterminée pour permettre in vivo la migration, la croissance et la multiplication des cellules nécessaires à la formation d'un tissu apte à assurer les fonctions d'un ligament naturel.

D'une manière importante, compte tenu du problème posé, de créer un support d'implantation de fibroblastes biocompatible et biodégradable, la matrice extracellulaire est préférentiellement composée de collagène, de chitosane et de glycosaminoglycanes.

Pour la préparation de cette matrice extracellulaire, on se reportera au document EP-A-0 296 078 dont le contenu est incorporé à la présente description en tant que de besoin.

Pour l'essentiel cette préparation s'effectue comme suit. On prépare une solution de collagène à laquelle on ajoute une solution de chitosane qui peut être obtenue par N-désacétylation de chitine extraite de carapaces de crustacés. On ajoute ensuite une solution de chondroïtines-sulfate. La solution visqueuse obtenue est ensuite coulée dans des moules appropriés aux utilisations envisagées, puis déshydratée. A noter que la déshydratation de ce mélange peut être obtenue de différentes façons en fonction des applications envisagées et de l'aspect souhaité.

Par exemple, en procédant à une lyophilisation, on obtient un mélange sous forme d'un feutre dont le diamètre moyen des pores peut varier de façon contrôlée en fonction des conditions opératoires, notamment de la proportion de matières sèches de la matrice extracellulaire liquide, de la vitesse et de la température de congélation. En soumettant le mélange à un simple séchage, on obtient un film. De même, si l'on précipite le mélange avec des solvants, on obtiendra une matrice extracellulaire sous forme d'une poudre, après broyage du précipité.

A partir de la technique de base de la préparation de la matrice extracellulaire, les constituants de celle-ci peuvent être rajoutés dans différentes proportions correspondant aux fourchettes précitées.

Par exemple, la matrice extracellulaire peut contenir entre 0,5 et 2 % de matières sèches constituées par les trois types de molécules précitées.

A titre d'exemple, la matrice extracellulaire liquide peut contenir environ 1,25 % de matières sèches constituées par 72 % de collagène, 20 % de chitosane et 8 % de glycosaminoglycanes.

Dans cet exemple, et suivant le protocole de préparation décrit ci-dessus, il est procédé de la façon suivante :

A une solution ou suspension de collagène (9 g dans 750 ml) que l'on agite pendant une heure environ et qu'on laisse reposer à une température de l'ordre de + 4°C pendant une durée de 18 heures environ, on ajoute en une seule fois une solution contenant environ 2,5 g de chitosane purifié dans 200 ml d'eau dont le pH est amené à 3,5 notamment par de l'acide acétique pur. Après agitation de cette solution pendant une heure environ, on ajoute, à faible débit, une solution contenant environ 1 g de glycosaminoglycanes dans approximativement 50 ml d'eau.

Le mélange obtenu est agité pendant une heure à une température comprise entre 20 et 25°C, puis éventuellement dégazé par ultrasons notamment. Il suffit ensuite de déshydrater le mélange.

Comme indiqué, les matrices extracellulaires peuvent contenir les constituants dans d'autres proportions que celles indiquées ci-dessus. Par exemple, en respectant le même protocole de préparation que celui qui vient d'être donné, les matrices extracellulaires peuvent contenir par exemple :

- soit 68 % de collagène, 25 % de chitosane et 7 % de glycosaminoglycanes
- soit 80 % de collagène, 12 % de chitosane et 8 % de glycosaminoglycanes.

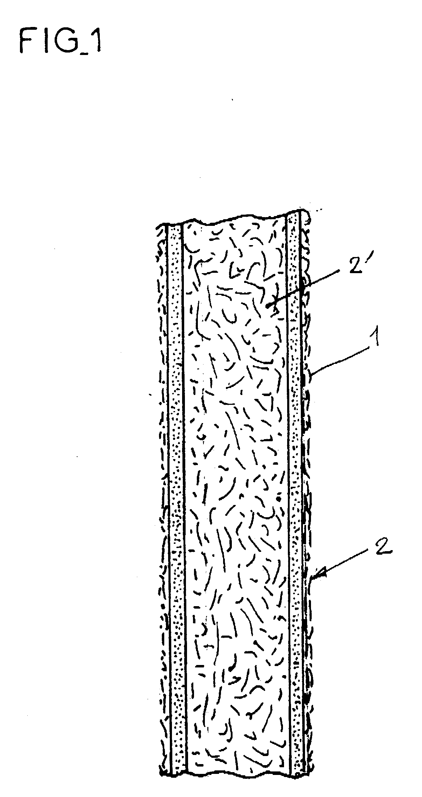

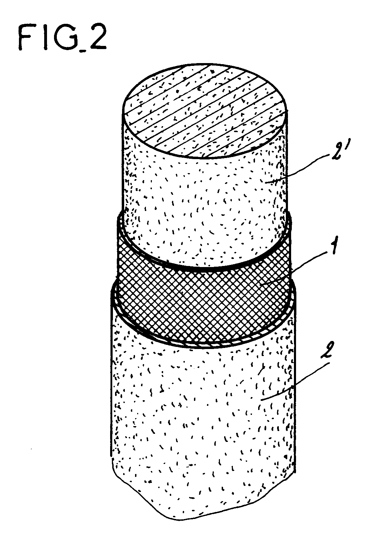

Selon les Figures 1 et 2, le support ou gaine 1, destiné à recevoir les matrices extracellulaires, telles que décrites précédemment, est du type tubulaire en étant conformé pour être implanté à la place du ou des ligaments naturels à remplacer. Bien évidemment, cette gaine 1 présente tout agencement pour sa mise en place et sa fixation au niveau de l'articulation considérée. La structure de la gaine 1 n'est pas décrite en détail, car elle ne fait pas partie de l'objet spécifique de l'invention. En effet, les matrices extracellulaires peuvent être combinées avec tout type de gaine biocompatible dont les formes, dimensions et agencements permettant de constituer un ligament artificiel.

La gaine 1 peut être obtenue par tissage ou tricotage de fibres de matériau biocompatible. Par exemple, la gaine est obtenue à partir d'un tissage de fibres en téréphtaéthylène-glycol, sans pour cela exclure tout autre polyester compatible susceptible d'être mis en forme par des caractéristiques similaires. La gaine est déterminée pour présenter les caractéristiques mécaniques notamment de résistance à la traction suffisante pendant le temps nécessaire à la reconstitution du néoligament sous l'effet de l'action de la matrice extracellulaire. De même, la nature du matériau constitutif de la gaine 1 est déterminée pour ne pas engendrer de réactions inflammatoires.

Concernant les caractéristiques mécaniques de la prothèse, des tests et essais de traction, notamment ont été effectués sur un appareil de mesure du type ADAMEL LHOMARGY DY 25.

Concernant la cytotoxicité de la prothèse stérilisée constituée par une gaine de polytéréphtaéthylène-glycol et de matrices extracellulaires constituées par l'association collagène,72 %-chitosane,20 %-chondroïtine-4-et-6-sulfate,8 %, une étude permet de confirmer la non cytotoxicité du ligament artificiel vis-à-vis des fibroblastes. L'évaluation de cette cytotoxicité a été faite quantitativement par un test au MTT en présence de matrice extracellulaire seule et matrice extracellulaire plus DACRON, comparativement à une culture de fibroblastes faite en présence d'un témoin de cytotoxicité positif et négatif.

| | Pourcentage de viabilité |
|---|---|
| Témoin de cytotoxicité négatif | 100 % |
| Témoin de cytotoxicité positif | 9 % |
| Matrice extracellulaire | 100 % |
| DACRON + Matrice extracellulaire test direct | 80 % |
| DACRON + Matrice extracellulaire test indirect | 92 % |

Le test direct consiste à déposer directement un fragment de DACRON + matrice extracellulaire sur une culture de fibroblastes. Pour le test indirect, on utilise le milieu de culture dans lequel a incubé 48 heures le produit à tester.

La coloration au MTT permet de transformer les sels de tétrazolium en cristaux bleus de formazan et dépend directement de l'activité de l'enzyme succinate déshydrogénase.

Les résultats obtenus sont satisfaisants : 20 % de cytotoxicité sont tolérés.

Concernant la colonisation des matrices extracellulaires de même nature que celle décrite ci-dessus, l'étude suivante a été réalisée :

La matrice extracellulaire a été ensemencée par des fibroblastes à raison de 500 000 cellules par cm$^2$ à $T_o$. La culture est incubée à 37°C dans une atmosphère à 5 % de $CO_2$ dans du milieu DMEM contenant 10 % de sérum de veau. Le milieu est changé deux fois par semaine. Les coupes histologiques sont faites à $T_o$ + 7 jours, 15 jours, 21 jours et 28 jours.

Les fibroblastes ont recolonisé la matrice extracellulaire de façon homogène à $T_o$ + 28 jours, comme montré à la Figure 4.

Chacun des deux procédés de réalisation est à présent décrit.

Pour la réalisation d'une prothèse ligamentaire de diamètre notamment inférieur à 9 mm, on procède en une seule étape.

La gaine 1 est introduite dans un tube ouvert à ses deux extrémités et dont le diamètre est supérieur d'environ 20 % au diamètre de ladite gaine. La matrice extracellulaire à l'état liquide est introduite par l'une des ouvertures du tube à un débit convenable pour assurer une imprégnation homogène des fibres de la gaine. L'ensemble est congelé comme indiqué précédemment puis soumis à une opération de déshydratation dans le tube ou après démoulage.

A noter que ce procédé peut également être utilisé dans certains cas pour la réalisation de ligaments de diamètre supérieur à 9 mm, mais ne permet pas de réaliser des ligaments présentant des différences au niveau de la composition et de la texture entre la matrice extracellulaire interne et la matrice extracellulaire externe.

A titre d'exemple, le diamètre extérieur de la gaine peut être de 4 à 5 mm, la longueur de la prothèse obtenue de 10 à 20 cm, sa résistance à la rupture de l'ordre de 120 daN et son allongement à la rupture de l'ordre de 24,4 %.

Pour la réalisation d'une prothèse ligamentaire d'un diamètre au moins égal à environ 9 mm, on procède en deux étapes.

Dans une première étape, après préparation de la matrice extracellulaire interne liquide, comme indiqué précédemment, cette dernière est coulée dans un tube cylindrique en matière plastique tel que le polyéthylène, polypropylène, polytétrafluorocarbone, silicone... On procède ensuite à une congélation de la matrice extracellulaire interne coulée dans le tube pendant un temps nécessaire à l'obtention d'une température stable au sein de ladite matrice extracellulaire. Cette dernière est alors déshydratée directement dans le tube de congélation ou après démoulage.

La matrice extracellulaire interne 2' déshydratée est ensuite introduite dans la gaine 1 constituant ainsi un corps cylindrique résultant de l'introduction de ladite matrice extracellulaire interne 2' à l'état sec à l'intérieur de ladite gaine 1.

Dans une deuxième étape, l'ensemble constitué de la gaine 1 et de la matrice extracellulaire interne 2' à l'état sec est introduit dans un tube contenant une solution de matrice extracellulaire externe à l'état liquide et en quantité suffisante pour entourer ledit ensemble dans sa totalité. Après une durée de l'ordre de 5 à 15 minutes, l'ensemble contenu dans le tube et imprégné de la matrice extracellulaire externe liquide est congelé dans les mêmes conditions que celles décrites précédemment. On procède ensuite à la déshydratation dans le tube ou après démoulage pour obtenir la prothèse ligamentaire.

A titre d'exemple, le diamètre extérieur du support peut être de 9 à 10 mm, la longueur de la prothèse obtenue de l'ordre de 25 à 30 cm, sa résistance à la rupture de l'ordre de 238 daN et son allongement de l'ordre de 28,4 %.

Ce procédé de préparation permet d'utiliser des compositions différentes de la matrice extracellulaire pour la matrice interne et la matrice externe.

## Revendications

1. Prothèse ligamentaire comprenant une armature allongée, souple et résistante, et une matrice extracellulaire couvrant ladite armature, <u>caractérisée en ce que</u> l'armature est une gaine (1) tubulaire perméable à la matrice extracellulaire à l'état liquide, ladite gaine étant remplie avec une matrice extracellulaire interne (2') sèche et recouverte à l'extérieur par une matrice extracellulaire externe (2) sèche, la gaine étant traversée par l'une et/ou l'autre des deux matrices extracellulaires.

2. Prothèse selon la revendication 1, caractérisée en ce que la gaine est constituée par des fils synthétiques tissés, tels que le polytéréphtaéthylène-glycol, tressés ou tricotés.

3. Prothèse selon la revendication 1, caractérisée en ce que les matrices extracellulaires interne et externe sont constituées par un matériau biologique extracellulaire artificiel.

4. Prothèse selon la revendication 3, caractérisée en ce que le matériau extracellulaire biologique artificiel est composé de collagène, chitosane et glycosaminoglycanes.

5. Prothèse selon la revendication 4, caractérisée en ce que le collagène est choisi parmi les collagènes de type I à XV d'origine humaine ou animale, natifs ou sans télopeptide et que sa concentration pondérale par rapport au poids sec de la matrice, est comprise entre 60 et 80 %.

6. Prothèse selon la revendication 4, caractérisée en ce que le chitosane provient de la N-désacétylation de la chitine d'invertébrés et que sa concentration pondérale par rapport au poids sec de la matrice est comprise entre 10 et 30 %.

7. Prothèse selon la revendication 4, caractérisée en ce que les glycosaminoglycanes sont choisis parmi l'acide hyaluronique, la chondroïtine-4-sulfate, la chondroïtine-6-sulfate, le dermatane-sulfate, l'héparane-sulfate, le kératane-sulfate et l'héparine, et que leur concentration pondérale par rapport au poids sec de la matrice, est comprise entre 6 et 10 %.

8. Prothèse selon la revendication 3, caractérisée en ce que le matériau extracellulaire biologique artificiel est choisi parmi les collagènes réticulés et dérivés, les chitosanes réacétylés et dérivés, les chitosanes réticulés et dérivés, la combinaison du collagène réticulé et du chitosane réticulé, le collagène réticulé avec du chitosane, et la combinaison du collagène réticulé et de glycosaminoglycanes réticulés.

9. Prothèse selon la revendication 3, caractérisée en ce que les compositions des matrices extracellulaires interne et externe sont les mêmes ou différentes.

10. Procédé pour réaliser une prothèse ligamentaire selon l'une quelconque des revendications 1 à 8, ayant un diamètre au moins égal à environ 9 mm, caractérisé en ce que :
    - après préparation des matrices extracellulaires liquides, on coule la matrice extracellulaire interne (2') dans un tube cylindrique et on la congèle pendant un temps déterminé apte à conférer une température stable au sein du matériau
    - on déshydrate la matrice extracellulaire interne (2') notamment par lyophilisation
    - on introduit la matrice extracellulaire interne (2') déshydratée dans la gaine
    - on plonge la gaine (1) contenant la matrice extracellulaire interne (2') déshydratée dans un tube

EP 0 454 599 B1

contenant une quantité de matrice extracellulaire externe (2) liquide pour imprégner ladite gaine (1) dans sa totalité et on congèle l'ensemble après un temps de contact déterminé
- on déshydrate l'ensemble notamment par lyophilisation.

11. Procédé pour réaliser une prothèse ligamentaire selon l'une quelconque des revendications 1 à 8, ayant un diamètre notamment inférieur à 9 mm, caractérisé en ce que :
    - on introduit la gaine (1) dans un tube ouvert à ses deux extrémités
    - on introduit les matrices extracellulaires interne (2')et externe (2) liquides par l'une des extrémités du tube pour assurer un remplissage et une imprégnation homogène de la gaine (1)
    - on congèle l'ensemble
    - on déshydrate l'ensemble, notamment par lyophilisation.

**Patentansprüche**

1. Bandprothese, mit einem länglichen flexiblen und strapazierfähigen Stützwerk, und mit einer das Stützwerk bedeckenden extrazellulären Matrix, <u>dadurch gekennzeichnet</u>, daß das Stützwerk als rohrförmige Hülle (1) ausgebildet ist, die für die sich im flüssigen Zustand befindliche extrazelluläre Matrix durchlässig ist, wobei die Hülle mit einer inneren trockenen extrazellulären Matrix (2') gefüllt ist und auf der Außenseite mit einer äußeren trockenen extrazellulären Matrix (2) bedeckt ist, wobei die Hülle von der einen und/oder der anderen der beiden extrazellulären Matrizes durchdrungen ist.

2. Prothese nach Anspruch 1, dadurch gekennzeichnet, daß die Hülle aus gewebten synthetischen Fäden, wie geflochtenes oder gewirktes polyethylenglykolterephthalat gebildet ist.

3. Prothese nach Anspruch 1, dadurch gekennzeichnet, daß die inneren und äußeren extrazellulären Matrizes aus einem künstlich dargestellten biologischen extrazellulären Material gebildet sind.

4. Prothese nach Anspruch 3, dadurch gekennzeichnet, daß das künstlich dargestellte biologische extrazelluläre Material aus Kollagen, Chitosan und Glykosaminoglykanen zusammengesetzt ist.

5. Prothese nach Anspruch 4, dadurch gekennzeichnet, daß das Kollagen ausgewählt aus den Kollagenen des Types I bis XV menschlichen oder tierischen Ursprungs, natürlicher Art oder ohne Telopeptid ist, und daß dessen Gewichtsanteil bezüglich des Trockengewichts der Matrix zwischen 60 und 80 % liegt.

6. Prothese nach Anspruch 4, dadurch gekennzeichnet, daß das Chitosan aus der N-Desacetylierung des Chitins von Wirbellosen stammt, und daß dessen Gewichtsanteil bezüglich des Trockengewichts der Matrix zwischen 10 und 30 % liegt.

7. Prothese nach Anspruch 4, dadurch gekennzeichnet, daß die Glykosaminoglykane ausgewählt sind aus Hyaluronsäure, Chondroitin-4-sulfat, Chondroitin-6-sulfat, Dermatansulfat, Heparansulfat, Keratansulfat und Heparin, und daß deren Gewichtsanteil bezüglich des Trockengewichts der Matrix zwischen 6 und 10 % liegt.

8. Prothese nach Anspruch 3, dadurch gekennzeichnet, daß das künstlich dargestellte biologische extrazelluläre Material ausgewählt ist aus den vernetzten und derivativen Kollagenen, den reacetylisierten und derivativen Chitosanen, den vernetzten und derivativen Chitosanen, der Kombination von vernetztem Kollagen und vernetztem Chitosan, von vernetztem Kollagen mit Chitosan, und der Kombination von vernetztem Kollagen und vernetzten Glykosaminoglykanen.

9. Prothese nach Anspruch 3, dadurch gekennzeichnet, daß die Zusammensetzungen der inneren und äußeren extrazellulären Matrizes gleich oder unterschiedlich sind.

10. Verfahren zum Herstellen einer Bandprothese nach einem der Ansprüche 1 bis 8, die einen Durchmesser von zumindest gleich bzw. etwa 9 mm aufweist, dadurch gekennzeichnet, daß
    - nach Herstellen der flüssigen extrazellulären Matrizes die innere extrazelluläre Matrix (2') in ein zylindrisches Rohr gegossen wird, und man diese während eines bestimmten Zeitraumes, der ausreichend ist, um das innere des Materials auf eine stabile Temperatur zu bringen, zum Erstarren bringt,
    - die innere extrazelluläre Matrix (2'), insbesondere durch Gefriertrocknen, dehydratisiert wird,

7

- die dehydratisierte innere extrazelluläre Matrix (2') in die Hülle eingeführt wird,
- die Hülle (1), die die dehydratisierte innere extrazelluläre Matrix (2') enthält, in ein Rohr getaucht wird, das eine Menge der flüssigen äußeren extrazellulären Matrix (2) enthält, um die Hülle (1) insgesamt zu imprägnieren, und das Gesamte nach einer bestimmten Kontaktzeit zum Erstarren gebracht wird,
- und das Gesamte, insbesondere durch Gefriertrocknen, dehydratisiert wird.

11. Verfahren zum Herstellen einer Bandprothese nach einem der Ansprüche 1 bis 8, die insbesondere einen Durchmesser unterhalb von 9 mm aufweist, dadurch gekennzeichnet, daß
- die Hülle (1) in ein an seinen beiden äußeren Enden offenes Rohr eingeführt wird,
- die flüssige innere extrazelluläre Matrix (2') und die flüssige äußere extrazelluläre Matrix (2) durch eines der Enden des Rohres eingebracht wird, so daß ein Füllen und ein homogenes Imprägnieren der Hülle (1) sichergestellt ist,
- das Gesamte zum Erstarren gebracht wird, und
- das Gesamte, insbesondere durch Gefriertrocken, dehydratisiert wird.

## Claims

1. Ligament prosthesis comprising an elongate, flexible and strong frame, and an extracellular matrix covering the said frame, <u>characterized in that</u> the frame is a tubular sheath (1) permeable to the extracellular matrix in the liquid state, the said sheath being filled with a dry inner extracellular matrix (2') and covered on the outside by a dry outer extracellular matrix (2), one and/or the other of the two extracellular matrices passing through the sheath.

2. Prosthesis according to Claim 1, characterized in that the sheath consists of woven synthetic threads, such as polyethylene terephthalate, braided or knitted.

3. Prosthesis according to Claim 1, characterized in that the inner and outer extracellular matrices consist of an artificial extracellular biological substance.

4. Prosthesis according to Claim 3, characterized in that the artificial biological extracellular substance is composed of collagen, chitosan and glycosaminoglycans.

5. Prosthesis according to Claim 4, characterized in that the collagen is chosen from among the collagens of type I to XV of human or animal origin, native or without telopeptide, and in that its concentration by weight, relative to the dry weight of the matrix, is between 60 and 80%.

6. Prosthesis according to Claim 4, characterized in that the chitosan is obtained by N-deacetylation of the chitin of invertebrates, and in that its concentration by weight, relative to the dry weight of the matrix, is between 10 and 30%.

7. Prosthesis according to Claim 4, characterized in that the glycosaminoglycans are chosen from among hyaluronic acid, chondroitin-4-sulphate, chondroitin-6-sulphate, dermatan sulphate, heparan sulphate, keratan sulphate and heparin, and in that their concentration by weight, relative to the dry weight of the matrix, is between 6 and 10%.

8. Prosthesis according to Claim 3, characterized in that the artificial biological extracellular substance is chosen from among the crosslinked collagens and derivatives, the reacetylated chitosans and derivatives, the crosslinked chitosans and derivatives, the combination of crosslinked collagen and crosslinked chitosan, crosslinked collagen with chitosan, and the combination of crosslinked collagen and crosslinked glycosaminoglycans.

9. Prosthesis according to Claim 3, characterized in that the compositions of the inner and outer extra-cellular matrices are the same or different.

10. Process for preparing a ligament prosthesis according to any one of Claims 1 to 8, having a diameter at least equal to approximately 9 mm, characterized in that:
- after preparation of the liquid extracellular matrices, the inner extracellular matrix (2') is poured into a cylindrical tube, and it is frozen for a defined period of time capable of conferring a stable temperature in the substance,

- the inner extracellular matrix (2') is dehydrated, in particular by lyophilization,
- the dehydrated inner extracellular matrix (2') is introduced into the sheath,
- the sheath (1) containing the dehydrated inner extracellular matrix (2') is immersed in a tube containing a quantity of liquid, outer extracellular matrix (2) in order to impregnate the said sheath (1) in its entirety, and the whole is frozen after a defined period of contact,
- the whole is dehydrated, in particular by lyophilization.

11. Process for preparing a ligament prosthesis according to any one of Claims 1 to 8, having a diameter which is in particular smaller than 9 mm, characterized in that:
    - the sheath (1) is introduced into a tube which is open at both ends,
    - the inner (2') and outer (2) liquid extra-cellular matrices are introduced via one of the ends of the tube in order to ensure a filling and a homogeneous impregnation of the sheath (1),
    - the whole is frozen,
    - the whole is dehydrated, in particular by lyophilization.

FIG_1

FIG_2

FIG.3

FIG.4